# EUROPÄISCHE PATENTSCHRIFT

(11) **EP 1 492 759 B1**
(45) Veröffentlichungstag und Bekanntmachung des Hinweises auf die Patenterteilung: **04.08.2010**
(21) Anmeldenummer: 03732270.8
(22) Anmeldetag: 29.03.2003
(51) Int. Cl.: C07C 69/67, C07C 69/003, C11B 9/00

(54) **ALICYCLISCHE ESTER MIT MOSCHUSGERUCH**
NOVEL ALICYCLIC ESTERS HAVING A MUSKY SMELL
NOUVEAUX ESTERS ALICYCLIQUES A ODEUR DE MUSC

(30) Priorität: 03.04.2002 DE 10214675
(43) Veröffentlichungstag der Anmeldung: 05.01.2005
(73) Patentinhaber: Symrise GmbH & Co. KG, 37603 Holzminden (DE)
(72) Erfinder: EH, Marcus, 37603 Holzminden (DE)
(74) Vertreter: Eisenführ, Speiser & Partner
(86) Internationale Anmeldenummer: PCT/EP2003/003294
(87) Internationale Veröffentlichungsnummer: WO 2003/082799

(56) Entgegenhaltungen:
- EP-A- 0 472 966
- WO-A-00/14051
- DE-A1- 2 650 602
- FR-A- 2 008 167
- US-A- 4 504 412
- DATABASE WPI Section Ch, Week 199545 Derwent Publications Ltd., London, GB; Class D21, AN 1995-348503 XP002251564 -& JP 07 238297 A (NIPPON OILS & FATS CO LTD), 12. September 1995 (1995-09-12)

## Beschreibung

Die Erfindung betrifft neue alicyclische Ester, Verfahren zu ihrer Herstellung, ihre Verwendung als Riechstoffe sowie parfümierte Produkte und Riechstoffmischungen enthaltend die erfindungsgemäßen Verbindungen.

Verbindungen mit Moschusgeruch sind begehrte Komponenten in der Duftstoffindustrie. Sie zeichnen sich sowohl durch ihre Eigenschaft, Parfümkompositionen Ausstrahlung zu verleihen, als auch durch ihre Fähigkeit, als Fixateur zu wirken, aus. Somit kommen heutzutage Moschus Riechstoffe in vielen Parfümkompositionen zum Einsatz.

Die Synthese von bioabbaubaren Verbindungen mit Moschusgeruch hat in den letzten Jahren stark an Bedeutung gewonnen, da die synthetischen Moschusverbindungen der nitroaromatischen und polycyclischen Reihe persistent und lipophil sind, so dass diese Verbindungen sich in aquatischen Nahrungsketten und Fettgewebe anreichern (H. Brunn, G. Rimkus, Emährungs-Umschau 1996, 43, 442 bis 449; H. Brunn, G. Rimkus, Ernährungs-Umschau 1997, 44, 4 bis 9). Um die Lücke zu schließen, wurden verstärkt naturähnliche makrocyclische Moschusriechstoffe entwickelt, welche sich durch einen makrocyclischen Ring mit 13 bis 17 C-Atomen auszeichnen, der als funktionelle Gruppe ein Keton oder einen Ester aufweist.

Weiterhin sind aus US-A 5,166,412 Verbindungen vom Typ (II) 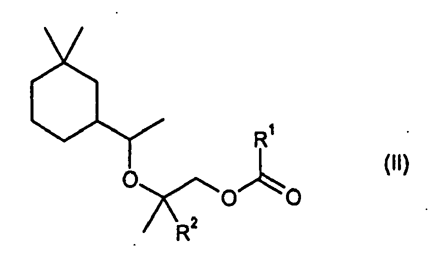 bekannt, worin R¹ eine C₁ bis C₃ Alkylgruppe und R² ein H oder eine Methylgruppe ist. Diese Verbindungen zeichnen sich durch einen Moschusgeruch aus, der mit ambrierten und fruchtigen Aspekten gepaart ist.

Darüber hinaus zeigt WO-A 00/14051, dass Ester vom Typ (III) 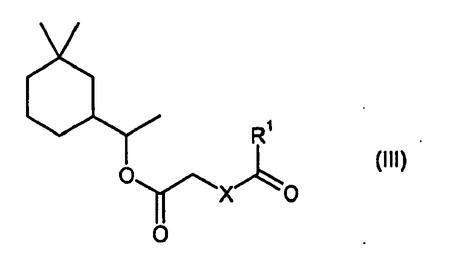 worin R¹ eine C₁ bis C₄ Alkyl- oder Alkylengruppe und X ein Sauerstoff, eine Methylen oder Ethylengruppe ist, ebenfalls Moschusgeruch aufweisen, wobei die ambrierten und fruchtigen Aspekte stärker in den Hintergrund treten.

Weitere Riechstoffe sind in den Dokumenten EP 0 472 966, FR 2 008 167, US 4,504,412, JP 07 238297 und DE 26 50 602 beschrieben.

Es stellte sich jetzt die Aufgabe, Verbindungen zu finden, die zum einen Moschusgeruch aufweisen und darüber hinaus mit weiteren originellen geruchlichen Aspekten, die für die Komposition von Parfüms zur Verfügung stehende Rohstoff-Palette erweitern.

Gegenstand der vorliegenden Erfindung sind neue alicyclische Ester der Formel (I) 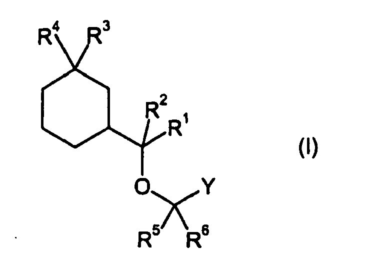 worin
i) R¹ = CH₃, R³ = H oder CH₃ und R² und R⁴ = H sind,
   R⁵ und R⁶ -unabhängig voneinander- H oder CH₃ sind, und
   Y = -CR⁷R⁸OCOR⁹, wobei R⁷und R⁸ -unabhängig voneinander- H oder CH₃ sind und
   - R⁹: eine verzweigte oder unverzweigte C₁ bis C₅ Alkylgruppe oder eine verzweigte oder unverzweigte C₂ bis C₅ Alkenylgruppe ist,
   oder
ii) R¹ und R² -unabhängig voneinander- CH₃ oder CH₂CH₃ sind,
   R³ und R⁴ -unabhängig voneinander- H oder CH₃ sind,
   R⁵ und R⁶ zusammen Sauerstoff sind, und
   Y=-CR⁷R⁸OCOR⁹, wobei R⁷, R⁸ und R⁹ die obengenannte Bedeutung haben
   oder
   iii) R¹ und R² -unabhängig voneinander- CH₃ oder CH₂CH₃ sind,
   R³, R⁴, R⁵ und R⁸ -unabhängig voneinander- H oder CH₃ sind, und
   Y = -CR⁷R⁸OCOR⁹ ist, wobei R⁷, R⁸ und R⁹ die oben genannte Bedeutung haben.

Ein weiterer Gegenstand der vorliegenden Erfindung sind Verfahren zur Herstellung der erfindungsgemäßen Verbindungen, deren Verwendung als Riechstoffe sowie parfümierte Produkte und Riechstoffmischungen enthaltend die erfindungsgemäßen Verbindungen.

Erfindungsgemäß werden unter verzweigten oder unverzweigten C₁ bis C₅ Alkylgruppen insbesondere die Alkylreste Methyl, Ethyl, n-Propyl, iso-Propyl, n-Butyl, sek.-Butyl, iso-Butyl, tert.-Butyl, n-Pentyl, iso-Pentyl und 3-Methylbutyl verstanden. Bevorzugt sind die Alkylreste Methyl, Ethyl, n-Propyl, iso-Propyl, n-Butyl, sek.-Butyl und iso-Butyl, besonders bevorzugt sind die Reste Methyl, Ethyl und n-Propyl.

Erfindungsgemäß werden unter verzweigten oder unverzweigten C₂ bis C₅ Alkenyl gruppen insbesondere die Alkenylreste Ethenyl, Methylethenyl, 1-Propenyl, 2-Propenyl, 2-Methyl-1-propenyl, 1-Methyl-1-propenyl, 1-Butenyl, 3-Butenyl, 1-Methyl-1-butenyl, 1-Methyl-3-butenyl, 3-Methyl-3-butenyl, 1-Pentenyl, 2-Pentenyl und 4-Pentenyl verstanden. Bevorzugt sind die Alkenylreste Ethenyl, Methylethenyl, 1-Propenyl, 2-Propenyl, 2-Methyl-1-propenyl, 1-Methyl-1-propenyl, besonders bevorzugt sind die Alkenylreste Ethenyl, Methylethenyl und 1-Propenyl.

Die neuen erfindungsgemäßen alicyclischen Ester der Formel (I) können in der optisch aktiven Form, wie auch als beliebige Mischungen ihrer Stereoisomere vorliegen.

Die erfindungsgemäßen alicyclischen Ester der Formel (I) erfüllen die gestellte Aufgabe, neben parfümistisch interessanten moschusartigen Geruchsnoten zeichnen sie sich durch interessante Beinoten aus. Hierbei wurde überraschenderweise gefunden, dass sich die Verbindungen, in denen R¹ und R² Methylgruppen sind, neben der Moschusnote durch sehr schöne holzige Aspekte auszeichnen. Die holzigen Aspekte treten vollständig in den Hintergrund, wenn R¹ eine Methylgruppe ist und R², R³ und R⁴ Wasserstoff sind, so dass sich diese Verbindungen durch blumige Aspekte, gepaart mit fruchtigen Akzenten auszeichnen.

Sensorisch besonders wertvoll sind Verbindungen der Formel (IV), die eine tertiäre Alkoxyfunktion besitzen (R¹, R² = CH₃), da sie neben der Moschusnote noch holzige Aspekte aufweisen. 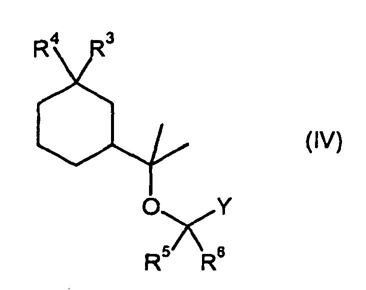 worin
R³ und R⁴ -unabhängig voneinander- H oder CH₃ sind, wobei R³ und R⁴ = Methyl bevorzugt ist
R⁵ und R⁶ zusammen Sauerstoff sind, und
Y = -CR⁷R⁸OCOR⁹ ist, wobei R⁷, R⁸ und R⁹ die oben genannte Bedeutung haben, wobei Y = Methyl, Ethyl oder n-Propyl, wie auch Y = -CR⁷R⁸OCOR⁹ mit R⁷ und R⁸ = H und R⁹ = Methyl, Ethyl oder n-Propyl bevorzugt ist.

Ganz besonders bevorzugt, aufgrund seiner attraktiven olfaktorischen Eigenschaften, ist die Verbindung der Formel (V), wobei sich der süße, erogene Moschusgeruch in einzigartiger Weise mit weichen holzigen Aspekten paart.

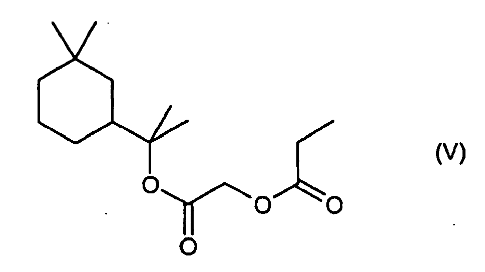

Eine weitere Klasse sensorisch sehr interessanter Moleküle, die neben der Moschusnote noch blumige und fruchtige Aspekte aufweisen, sind in Formel (VI) dargestellt. 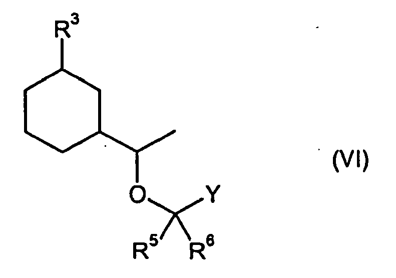 worin
R³ = H oder CH₃ ist,
R⁵ und R⁶ -unabhängig voneinander- H oder CH₃ sind, wobei R⁵, R⁶ = Methyl bevorzugt ist, und
Y = -CR⁷R⁸OCOR⁹ ist, wobei R⁷, R⁸ und R⁹ die vorgenannte Bedeutung haben, wobei R⁷ und R⁸ = H und R⁹ = Methyl, Ethyl oder n-Propyl bevorzugt ist.

Insbesondere bevorzugt, aufgrund ihrer attraktiven olfaktorischen Eigenschaften sind die Isomere der Formeln (VIIa) und (VIIb), wobei sich der erogene, leicht ambrierte Moschusgeruch in einzigartiger Weise mit intensiven blumigen Aspekten paart.

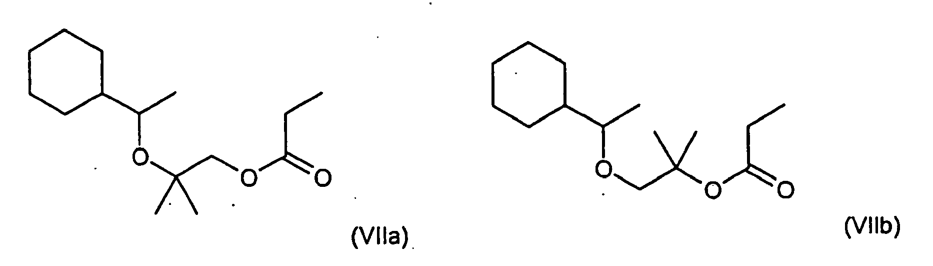

Die erfindungsgemäßen alicyclischen Ester der Formel (I) können dabei als Einzelstoffe in einer Vielzahl von Produkten verwendet werden; besonders vorteilhaft lassen sie sich mit anderen Riechstoffen zu neuartigen Parfümkompositionen kombinieren.

Durch die Verwendung der erfindungsgemäßen alicyclischen Ester der Formel (I) lassen sich in der Regel bereits in geringer Dosierung in den resultierenden Parfümkompositionen feine, erogene Moschusnoten, gepaart mit holzigen oder blumigen Akzenten erzielen, wobei der geruchliche Gesamteindruck auffallend harmonisiert, die Ausstrahlung wahrnehmbar erhöht und die Fixierung, d.h. das Haftvermögen des Parfümöles, deutlich verstärkt wird.

Beispiele für Riechstoffe, mit denen die erfindungsgemäßen alicyclischen Ester der Formel (I) vorteilhaft kombiniert werden können, finden sich z.B. in K. Bauer, D. Garbe und H. Surburg, Common Fragrance and Flavor Materials, 3rd. Ed., Wiley-VCH, Weinheim 1997.

Im einzelnen seien genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedemholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z.B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren 1,4-Dioxacycloalken-2-one wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-l-yl)-2-buten-l-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on, 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 5-Cyclohexadecen-1-on; 8-Cyclohexadecen-1-on; 9-Cycloheptadecen-1-on; Cyclopentadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)-propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)-propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die die erfindungsgemäßen alicyclischen Ester der Formel (I) enthaltenden Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können die die erfindungsgemäßen alicyclischen Ester der Formel (I) enthaltenden Parfümöle an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die die erfindungsgemäßen alicyclischen Ester der Formel (I) enthaltenden Parfümöle können auch mikroverkapselt, sprühgetrocknet, als Einschluss-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus Polyurethanartigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

In Parfümkompositionen beträgt die eingesetzte Menge der erfindungsgemäßen alicyclischen Ester der Formel (I) 0,05 bis 50 Gew.-%, vorzugsweise 0,5 bis 20 Gew.-%, bezogen auf das gesamte Parfümöl.

Die die erfindungsgemäßen alicyclischen Ester der Formel (I) enthaltenden Parfümöle können in konzentrierter Form, in Lösungen oder in oben beschriebener modifizierter Form verwendet werden für die Herstellung von z.B. Parfüm-Extraits, Eau de Parfüms, Eau de Toilettes, Rasierwässer, Eau de Colognes, Pre-shave-Produkte, Splash-Colognes und parfümierten Erfrischungstüchem sowie die Parfümierung von sauren, alkalischen und neutralen Reinigungsmitteln, wie z.B. Fußbodenreinigern, Fensterglasreinigern, Geschirrspülmittel, Bad- und Sanitärreinigem, Scheuermilch, festen und flüssigen WC-Reinigem, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserem in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und -lotionen, After-shave-Cremes und - lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Alicyclische Ester, unter anderem erfindungsgemäße Ester, können beispielsweise folgendermaßen hergestellt werden:

Die Herstellung der erfindungsgemäßen alicyclischen Ester der Formel (I), worin
R¹ und R² -unabhängig voneinander- CH₃ oder CH₂CH₃ sind,
R³ und R⁴ -unabhängig voneinander- H oder CH₃ sind,
R⁵ und R⁶ zusammen Sauerstoff sind, und
Y -CR⁷R⁸OCOR⁹ ist, wobei R⁷und R⁸ -unabhängig voneinander- H oder CH₃ sind und
- R⁹: eine verzweigte oder unverzweigte C₁ bis C₅ Alkylgruppe oder eine verzweigte oder unverzweigte C₂ bis C₅ Alkylengruppe ist
gelingt nach Syntheseweg A.

In Syntheseweg A wird das substituierte Cyclohexylalkanol (VIII) mit der Carbonsäure (IX), welche durch Umsetzung der entsprechenden α-Hydroxycarbonsäure mit dem korrespondierenden Säurechlorid (Thayer, F.K., Organic Synthesis Col. Vol. 1 (1932), S. 12) erhältlich ist, verestert. Die Veresterung gelingt nach dem Fachmann wohlvertrauten Methoden, beispielsweise durch Erhitzen der beiden Edukte am Wasserabscheider in Gegenwart eines Schleppmittels (z.B. Toluol oder Cyclohexan) unter Zugabe von 0,01 Mol-% bis 10 Mol-%, bevorzugt 0,1 Mol-% bis 5 Mol-% einer Säure, bevorzugt p-Toluolsulfonsäure oder Schwefelsäure oder nach der sogenannten Steglich Methode, wobei unter Zugabe von Dicyclohexylcarbodiimid und 0,02 Mol-% bis 20 Mol-%, bevorzugt 0,5 Mol-% bis 10 Mol-% 4-Dimethylaminopyridin verestert wird.

Die Herstellung von alicyclischen Estern (nicht erfindungsgemäß) der Formel (I), worin
R¹ und R² -unabhängig voneinander- CH₃ oder CH₂CH₃ sind,
R³ und R⁴ -unabhängig voneinander- H oder CH₃ sind,
R⁵ und R⁶ zusammen Sauerstoff sind, und
Y = R⁹ ist und R⁹ die oben genannte Bedeutung hat,
gelingt nach Syntheseweg B.

In Syntheseweg B wird das substituierte Cyclohexylalkanol (VIII) nach dem Fachmann wohlvertrauten Methoden verestert. Hierbei kann die Veresterung durch Erhitzen des substituierten Cyclohexylalkanols (VIII) und der entsprechenden Carbonsäure am Wasserabscheider in Gegenwart eines Schleppmittels (z.B. Toluol oder Cyclohexan) unter Zugabe von 0,01 Mol-% bis 10 Mol-%, bevorzugt 0,1 Mol-% bis 5 Mol-% einer Säure, bevorzugt p-Toluolsulfonsäure oder Schwefelsäure erfolgen oder durch Umsetzung des substituierten Cyclohexylalkanols (VIII) mit dem entsprechenden Carbonsäureanhydrid in Gegenwart von Triethylamin und 0,5 Mol-% bis 50 Mol-%, bevorzugt 1,0 Mol-% bis 30 Mol-%, 4-Dimethylaminopyridin.

Die Herstellung der erfindungsgemäßen alicyclischen Ester der Formel (I), worin
R¹ = CH₃, R³ = H oder CH₃ und R² und R⁴ = H sind,
R⁵ und R⁶ -unabhängig voneinander- H oder CH₃ sind, und
Y = -CR⁷R⁸OCOR⁹, wobei R⁷, R⁸ und R⁹ die oben genannte Bedeutung haben,
oder
R¹ und R² -unabhängig voneinander- CH₃ oder CH₂CH₃ sind,
R³, R⁴, R⁵ und R⁶ -unabhängig voneinander- H oder CH₃ sind, und
Y = -CR⁷R⁸OCOR⁹ ist, wobei R⁷, R⁸ und R⁹ die oben genannte Bedeutung haben,
gelingt nach Syntheseweg C.

Im 1. Schritt von Syntheseweg C wird das Epoxid (X) nucleophil mit dem substituierten Cyclohexylalkanol (VIII) geöffnet. Wird bei der Umsetzung ein unsymmetrisch substituiertes Epoxid verwendet, so kann der resultierende Alkohol (XI) als Mischung zweier Regioisomere erhalten werden. Diese Umsetzung kann beispielsweise unter Zugabe von 0,02 Mol-% bis 20 Mol-%, bevorzugt 0,5 Mol-% bis 10 Mol-%, einer Lewissäure erfolgen, bevorzugte Lewissäuren enthalten ein Bor-Atom, insbesondere bevorzugt ist BF₃-OEt₂. Der resultierende Alkohol (XI) wird nach dem Fachmann wohlvertrauten Methoden verestert. Hierbei kann die Veresterung durch Erhitzen des Alkohols (XI) und der entsprechenden Carbonsäure am Wasserabscheider in Gegenwart eines Schleppmittels (z.B. Toluol oder Cyclohexan) unter Zugabe von 0,01 Mol-% bis 10 Mol-%, bevorzugt 0,1 Mol-% bis 5 Mol-% einer Säure, bevorzugt p-Toluolsulfonsäure oder Schwefelsäure erfolgen oder durch Umsetzung des Alkohols (XI) mit dem entsprechenden Carbonsäureanhydrid in Gegenwart von Triethylamin und 0,5 Mol-% bis 50 Mol-%, bevorzugt 1,0 Mol-% bis 30 Mol-%, 4-Dimethylaminopyridin.

Folgendes Formelschema kann die ggf. erfindungsgemäßen Verfahren erläutern:

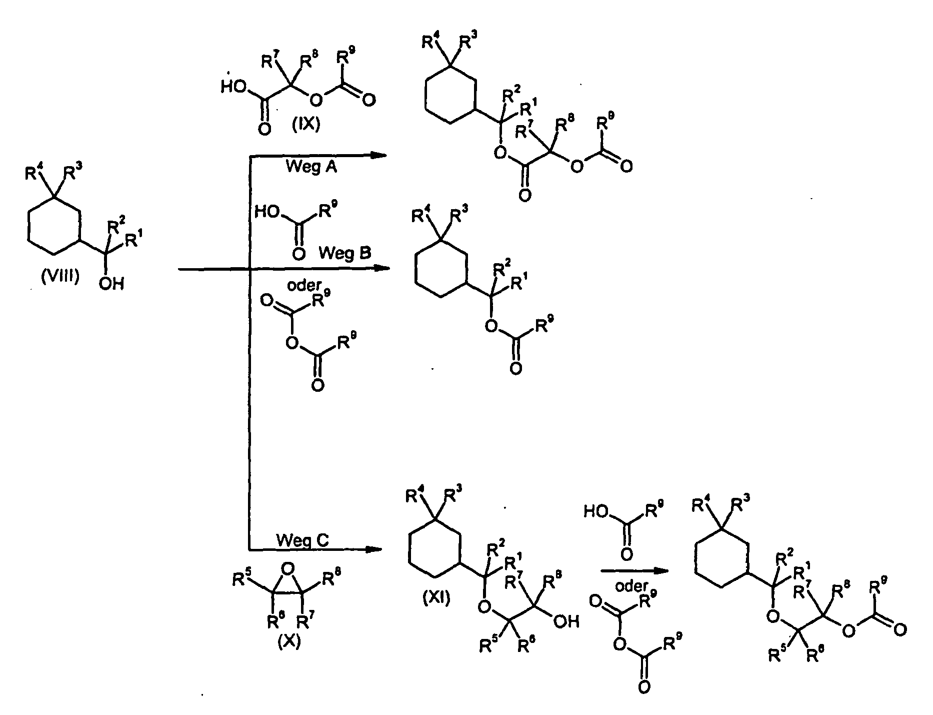

Alternativ können die erfindungsgemäßen alicyclischen Ester der Formel (I), worin
R¹ und R² -unabhängig voneinander- CH₃ oder CH₂CH₃ sind,
R³ und R⁴ -unabhängig voneinander- H oder CH₃ sind,
R⁵ und R⁶ zusammen Sauerstoff sind, und
Y= -CR⁷R⁸OCOR⁹ ist, wobei R⁷, R⁸ und R⁹ die oben genannte Bedeutung haben,
nach Syntheseweg D hergestellt werden.

Im 1. Schritt von Syntheseweg D wird das substituierte Cyclohexylalkanol (VIII) nach dem Fachmann wohlvertrauten Methoden verestert. Hierbei kann die Veresterung durch Erhitzen des substituierten Cyclohexylalkanols (VIII) und der entsprechenden Carbonsäure, wobei X eine OH-Gruppe oder ein Halogen, bevorzugt eine OH-Gruppe oder ein Chloratom ist, am Wasserabscheider in Gegenwart eines Schleppmittels (z.B. Toluol oder Cyclohexan) unter Zugabe von 0,01 Mol-% bis 10 Mol-%, bevorzugt 0,1 Mol-% bis 5 Mol-%, einer Säure, bevorzugt p-Toluolsulfonsäure oder Schwefelsäure, erfolgen. Des weiteren kann die Umsetzung des substituierten Cyclohexylalkanols (VIII) mit dem entsprechenden Carbonsäureanhydrid, wobei X eine OH-Gruppe oder ein Halogen, bevorzugt eine OH-Gruppe oder ein Chloratom ist, in Pyridin erfolgen.

Im 2. Schritt wird der Ester (XII) für den Fall das X = OH ist, mit der entsprechenden Carbonsäure (Z = H) verestert. Diese Umsetzung kann beispielsweise am Wasserabscheider in Gegenwart eines Schleppmittels (z.B. Toluol oder Cyclohexan) unter Zugabe von 0,01 Mol-% bis 10 Mol-%, bevorzugt 0,1 Mol-% bis 5 Mol-%, einer Säure erfolgen. Bevorzugte Säuren sind p-Toluolsulfonsäure oder Schwefelsäure. Des weiteren kann die Umsetzung des Esters (XII) mit X = OH mit dem entsprechenden Carbonsäureanhydrid (Z = -C(O)R⁹) in Gegenwart von Triethylamin und 0,5 Mol-% bis 50 Mol-%, bevorzugt 1,0 Mol-% bis 30 Mol-%, 4-Dimethylaminopyridin erfolgen.

Für den Fall X = Halogen, und bevorzugt X = Chlor, kann der Ester (XII) einerseits mit einem Alkalimetallsalz einer Carbonsäure (Z = Alkalimetall, bevorzugt sind Natrium und Kalium) in Gegenwart eines Alkalimetallsalzes, bevorzugt Natriumbromid, oder in Gegenwart des korrespondierenden Carbonsäureanhydrids umgesetzt werden. Andererseits kann der Ester (XII) mit X = Halogen und bevorzugt X = Chlor mit einer Carbonsäure (Z = H) in Gegenwart einer Base, bevorzugt Kaliumcarbonat, umgesetzt werden.

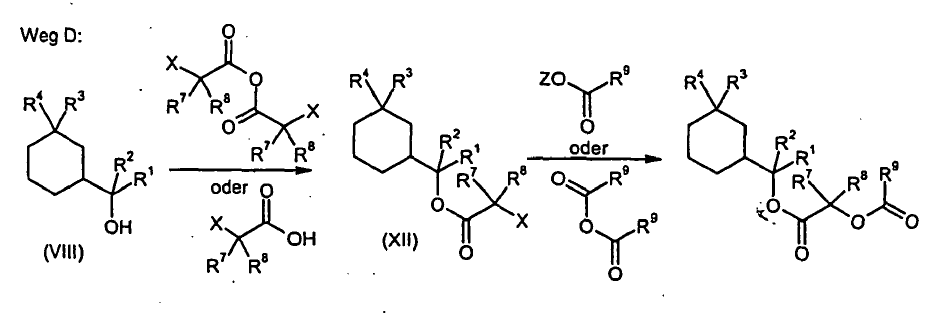

Die folgenden Beispiele erläutern die Erfindung:

### Beispiel 1

### Propionsäure-2-(1-cyclohexytethoxy)-2-methylpropylester

2-(1-Cyclohexylethoxy)-2-methyl-1-propanol: Zu einer auf 0°C abgekühlten Lösung von 1-Cyclohexylethanol (16,6 g, 127.0 mmol) und Isobutylenoxid (2,9 g, 40,0 mmol) in Cyclohexan (20 ml), tropft man BF₃-OEt₂ (2.0 ml). Jetzt lässt man bei 0°C weitere 30 Minuten rühren und gibt anschließend weiteres BF₃-OEt₂ (2,0 ml) hinzu. Nach weiteren 3 Stunden bei 0°C nimmt man die Kühlung weg und wäscht die Reaktionslösung einmal mit 1 M NaOH (15 ml). Die organische Phase wird über Na₂SO₄ getrocknet, abfiltriert und am Rotationsverdampfer von Lösungsmittel befreit. Von dem so erhaltenen Rohprodukt wird mittels Kugelrohrdestillation (KRD) das nicht abreagierte 1-Cyclohexylethanol abgenommen und man erhält 5,3 g rohes 2-(1-Cyclohexylethoxy)-2-methyl-1-propanol mit einem GC-Gehalt von 75 %, welches ohne weitere Reinigung in die nächste Reaktion eingesetzt werden kann.

Propionsäure-2-(1-cyclohexylethoxy)-2-methylpropylester: Zu einer Lösung aus 2-(1-Cyclohexylethoxy)-2-methyl-1-propanol (GC-Reinheit: 75 %; Rohprodukt aus der ersten Stufe) (1.46 g, 5,5 mmol) und Propionsäureanhydrid (2,0 g, 15 mmol) gibt man nacheinander Triethylamin (1,6 g, 15 mmol) und 4-Dimethylaminopyridin (0,13 g, 1,0 mmol). Nach einer Stunde rühren bei Raumtemperatur verdünnt man die Reaktionslösung mit Ether (100 ml) und wäscht die organische Phase je zweimal mit 2 M HCl und gesättigter NaHCO₃-Lösung. Die vereinigten organischen Phasen werden über Na₂SO₄ getrocknet, abfiltriert und am Rotationsverdampfer von Lösungsmittel befreit. Nach Flashchromatographie (Cyclohexan/EtOAc = 45:1, R_{f} = 0,25) und Kugelrohrdestillation (KRD: 137°C, 0,5 mbar) erhält man 930 mg (70 %) Propionsäure-2-(1-cyclohexylethoxy)-2-methylpropylester als farbloses Öl.
Geruch: stark moschus, erogen, ambriert, blumig.

¹H-NMR (200 MHz, CDCl₃): δ (ppm) = 0,80-1,35 (m, 6H), 1,06 (d, J = 6,1 Hz, 3H), 1,16 (t, J = 7,5 Hz, 3H), 1,18 (s, 6H), 1,55-1,85 (m, 5H), 2,37 (q, J = 7,5 Hz, 2H), 3,40 (quin, J = 6,1 Hz, 1H), 3,95 (s, 2H).
¹³C-NMR (50 MHz, CDCl₃): δ (ppm) = 9,1, 19,7, 23,8, 24,0, 26,5, 26,6, 26,7, 27,7, 28,5, 29,5, 44,9, 70,3, 71,6, 73,7, 174,3.

Die nachfolgenden Verbindungen der Beispiele 2 und 3 wurden analog der unter Beispiel 1 beschriebenen Vorschriften hergestellt, mit der Änderung, dass die Veresterung mit Essigsäureanhydrid (Beispiel 2) oder Isobuttersäureanhydrid (Beispiel 3) durchgeführt wurde. Somit sind an dieser Stelle nur die spektroskopischen Daten aufgeführt:

### Beispiel 2

### Essigsäure-2-(1-cyclohexylethoxy)-2-methylpropylester

Geruch: moschus, blumig, fruchtig.
KRD: 140°C, 0,6 mbar.
¹H-NMR (200 MHz, CDCl₃): δ (ppm) = 0,80-1,35 (m, 6H), 1,06 (d, J = 6,2 Hz, 3H), 1,18 (s, 6H), 1,56-1,88 (m, 5H), 2,08 (s, 3H), 3,40 (quin, J = 6,2 Hz, 1H), 3,92 (d, J = 11,2 Hz, 1H), 3,95 (d, J = 11,2 Hz, 1H).
¹³C-NMR (50 MHz, CDCl₃): δ (ppm) = 19,7, 20,9, 23,7, 24,0, 26,4, 26,5, 26,6, 28,4, 29,4, 44,8, 70,4, 71,6, 73,6, 170,8.

### Beispiel 3

### Isobuttersäure-2-(1-cyclohexylethoxy)-2-methylpropylester

Geruch: moschus, erogen, blumig.
KRD: 164°C, 0,5 mbar.
¹H-NMR (200 MHz, CDCl₃): δ (ppm) = 0,80-1,30 (m, 6H), 1,06 (d, J = 6,2 Hz, 3H), 1,18 (s, 6H), 1,19 (d, J = 7,0 Hz, 6H), 1,60-1,90 (m, 5H), 2,58 (hep, J = 7,0 Hz, 1H), 3,40 (quin, J = 6,2 Hz, 1H), 3,93 (s, 2H).
¹³C-NMR (50 MHz, CDCl₃): δ (ppm) =18,9 (2C), 19,7, 23,7, 24,0, 26,4, 26,5, 26,6, 28,4, 29,4, 34,1, 44,8, 70,1, 71,6, 73,7, 176,7.

Die nachfolgenden Verbindungen der Beispiele 4 und 5 wurden analog der unter Beispiel 1 beschriebenen Vorschriften hergestellt, mit der Änderung, dass als Alkoholkomponente 1-Cyclohexyl-1-propanol, anstatt 1-Cyclohexyl-1-ethanol eingesetzt wurde, welche durch Grignard Reaktion aus Cyclohexylmagnesiumchlorid und Propanal herstellbar ist. Die Veresterung wurde mit Essigsäureanhydrid (Beispiel 4) oder Propionsäureanhydrid (Beispiel 5) durchgeführt. Somit sind an dieser Stelle nur die spektroskopischen Daten aufgeführt:

### Beispiel 4

### Essigsäure-2-(1-cyclohexylpropoxy)-2-methylpropylester

Geruch: schwach moschus, blumig, fruchtig.
KRD: 161°C, 0,75 mbar.
¹H-NMR (200 MHz, CDCl₃): δ (ppm) = 0,80-1,30 (m, 6H), 0,86 (t, J = 7,4 Hz, 3H), 1,19 (s, 6H), 1,35-1,50 (m, 2H), 1,55-1,80 (m, 5H), 2,08 (s, 3H), 3,24 (q, J = 5,4 Hz, 1H), 3,95 (s, 2H).
¹³C-NMR (50 MHz, CDCl₃): δ (ppm) = 9,5, 20,9, 24,0, 24,1, 24,7, 26,6 (2C), 26,7, 28,5, 29,2, 41,5, 70,8, 73,5, 76,7, 170,9.

### Beispiel 5

### Propionsäure-2-(1-cyclohexylpropoxy)-2-methylpropylester

Geruch: moschus, blumig, fruchtig.
KRD: 155°C, 0,55 mbar.
¹H-NMR (200 MHz, CDCl₃): δ (ppm) = 0,86 (t, 7,4 Hz, 3H), 0,90-1,25 (m, 6H), 1,16 (t, J = 7,6 Hz, 3H), 1,18 (s, 6H), 1,30-1,55 (m, 2H), 1,55-1,85 (m, 5H), 2,36 (q, J = 7,6 Hz, 2H), 3,24 (q, J = 5,3 Hz, 1H), 3,94 (s, 2H).
¹³C-NMR (50 MHz, CDCl₃): δ (ppm) = 9,1, 9,5, 24,0, 24,1, 24,7, 26,6 (2C), 26,7, 27,7, 28,6, 29,2, 41,5, 70,6, 73,7, 76,7, 174,2.

Die nachfolgenden Verbindungen der Beispiele 6 und 7 wurden analog der unter Beispiel 1 beschriebenen Vorschriften hergestellt, mit der Änderung das als Alkoholkomponente 2-Cyclohexyl-2-propanol, anstatt 1-Cyclohexyl-1-ethanol eingesetzt wurde, welche durch Grignard Reaktion aus Cyclohexylmagnesiumchlorid und Aceton herstellbar ist. Weiterhin wurde Propylenoxid an Stelle von Isobutylenoxid eingesetzt. Die Veresterung wurde mit Essigsäureanhydrid (Beispiel 6) oder Propionsäureanhydrid (Beispiel 7) durchgeführt. Somit sind an dieser Stelle nur die spektroskopischen Daten aufgeführt:

### Beispiel 6

### Essigsäure-2-(1-cydohexyl-1-methylethoxy)propylester/Essigsäure-2-(1-cyclohexyl-1-methylethoxy)-1-methylethylester

Regioisomerenverhältnis = 1:3
Geruch: schwach moschus, fruchtig.
KRD: 142°C, 0,6 mbar.
Die spektroskopischen Angaben beziehen sich auf das Hauptisomere:
¹H-NMR (200 MHz, CDCl₃): δ (ppm) = 0,9-1,25 (m, 6H), 1,06 (s, 6H), 1,22 (d, J = 6,4 Hz, 3H), 1,70-1,85 (m, 5H), 2,03 (s, 3H), 3,28 (dd, J = 9,7, 5,0 Hz, 1H), 3,37 (dd, J = 9,7, 5,8 Hz, 1H), 5,00 (ddq, J = 5,0, 5,8, 6,4 Hz, 1H).
¹³C-NMR (50 MHz, CDCl₃): δ (ppm) = 16,9, 21,3, 22,5, 22,7, 26,7, 26,8, 26,9, 27,4, 27,5, 46,4, 63,4, 70,3, 78,0, 170,6.

### Beispiel 7

### Propionsäure-2-(1-cyclohexyl-1-methylethoxy)propylester/Propionsäure-2-(1-cyclohexyl-1-methylethoxy)-1-methylethylester

Regioisomerenverhältnis = 1:3
Geruch: moschus, fruchtig.
KRD: 152°C, 0,72 mbar.
Die spektroskopischen Angaben beziehen sich auf das Hauptisomere:
¹H-NMR (200 MHz, CDCl₃): δ (ppm) = 0,88-1,24 (m, 6H), 1,06 (s, 6H), 1,13 (t, J = 7,6 Hz, 3H), 1,23 (d, J = 6,4 Hz, 3H), 1,70-1,85 (m, 5H), 2,30 (q, J = 7,6 Hz, 2H), 3,28 (dd, J = 9,6, 5,1 Hz, 1H), 3,37 (dd, J = 9,6, 5,9 Hz, 1H), 4,97 (ddq, J = 5,1, 5,9, 6,4 Hz, 1H).
¹³C-NMR (50 MHz, CDCl₃): δ (ppm) = 9,2, 17,0, 22,5, 22,7, 26,7, 26,8, 26,9, 27,4, 27,5, 27,9, 46,5, 63,5, 70,1, 77,0, 174,1.

Die nachfolgenden Verbindungen der Beispiele 8 und 9 wurden analog der unter Beispiel 1 beschriebenen Vorschriften hergestellt, mit der Änderung das als Alkoholkomponente 1-(3-Methyl-cyclohexyl)-ethanol, anstatt 1-Cyclohexyl-1-ethanol eingesetzt wurde, welche durch Hydrierung von 3-Methylacetophenon herstellbar ist. Die Veresterung wurde mit Essigsäureanhydrid (Beispiel 8) oder Propionsäureanhydrid (Beispiel 9) durchgeführt. Somit sind an dieser Stelle nur die spektroskopischen Daten aufgeführt:

### Beispiel 8:

### Essigsäure-2-[1-(3-methyl-cyclohexyl)-ethoxy]-2-methylpropylester

Geruch: moschus, blumig, fruchtig.
KRD: 142°C, 0,75 mbar.
¹H-NMR (200 MHz, CDCl₃): δ (ppm) = 0,55 (q, J = 12,1 Hz, 1H), 0,72-0,86 (m, 1H), 0,88 (d, J = 6,6 Hz, 3H), 1,07 (d, J = 6,1 Hz, 3H), 1,18 (s, 6H), 1,20-1,40 (m, 4H), 1,60-1,82 (m, 4H), 2,08 (s, 3H), 3,40 (quin, J = 6,1 Hz, 1H), 3,95 (d, J = 11,2 Hz, 2H).
¹³C-NMR (50 MHz, CDCl₃): δ (ppm): 19,7, 20,9, 23,1, 23,8, 24,0, 26,2, 28,0, 32,7, 35,3, 35,4, 44,9, 70,5, 71,6, 73,7, 170,9.

### Beispiel 9:

### Propionsäure-2-[1-(3-methyl-cyclohexyl)-ethoxy]-2-methylpropylester

Geruch: stark moschus, blumig, fruchtig.
KRD: 153°C, 0,75 mbar.
¹H-NMR (200 MHz, CDCl₃): δ (ppm) = 0,55 (q, J = 12,1 Hz, 1H), 0,72-0,86 (m, 1H), 0,88 (d, J = 6,6 Hz, 3H), 1,07 (d, J = 6,1 Hz, 3H), 1,16 (t, J = 7,6 Hz, 3H), 1,18 (s, 6H), 1,20-1,40 (m, 4H), 1,60-1,82 (m, 4H), 2,37 (q, J = 7,6 Hz, 2H), 3,40 (quin, J = 6,1 Hz, 1H), 3,94 (d, J = 11,2 Hz, 2H).
¹³C-NMR (50 MHz, CDCl₃): δ (ppm): 9,1, 19,7, 23,0, 23,8, 24,1, 26,3, 27,7, 29,0, 32,7, 35,4, 37,1, 44,9, 70,3, 71,6, 73,8, 174,2.

### Beispiel 10a:

### Propionsäure-2-[1-(3,3-dimethylcyclohexyl)-1-methylethoxy]-2-oxoethylester

(Propionyloxy)essigsäure: Man erhitzt eine Lösung bestehend aus Propionsäurechlorid (64,7 g, 0,7 mol) und Hydroxyessigsäure (19,0 g, 0,25 mol) auf 40°C bis sich die Hydroxyessigsäure vollständig gelöst hat. Anschließend destilliert man den Überschuss Propionsäurechlorid ab und erhält 35,6 g Rohprodukt mit einem Gehalt von 82 % von (Propionyloxy)essigsäure als farblose Flüssigkeit. Das Rohprodukt kann ohne weitere Reinigung in die folgende Reaktion eingesetzt werden.

Propionsäure-2-[1-(3,3-dimethylcyclohexyl)-1-methylethoxy]-2-oxoethylester: Zu einer Lösung aus (Propionyloxy)essigsäure 81 % (2,7 g, 16,5 mmol) in CH₂Cl₂ (15 ml) werden nacheinander 4-Dimethylaminopyridin (122 mg, 1,0 mmol) und 2-(3,3-Dimethylcyclohexyl)-2-propanol (2,6 g, 15 mmol) [welches durch Grignard Reaktion aus Methylmagnesiumchlorid und 1-(3,3-Dimethylcyclohexyl)-ethanon erhältlich ist] zugegeben. Jetzt kühlt man die Reaktionslösung auf 0°C ab und gibt Dicyclohexylcarbodiimid (3,4 g, 16,5 mmol), gelöst in CH₂Cl₂ (5 ml), hinzu. Nach 1 h bei 0°C wird die Kühlung entfernt und man rührt noch 16 h bei Raumtemperatur weiter. Nachfolgend filtriert man den ausgefallenen Niederschlag ab und befreit das Filtrat am Rotationsverdampfer von Lösungsmittel. Das erhaltene Rohprodukt wird in n-Pentan (20 ml) aufgenommen und der sich bildende Niederschlag wird erneut abfiltriert. Das Filtrat wird noch je zweimal mit 0,5 M HCl und gesättigter NaHCO₃-Lösung gewaschen, anschließend wird die organische Phase über Na₂SO₄ getrocknet, abfiltriert und am Rotationsverdampfer eingeengt. Nach flashchromatographischer Reinigung (Cyclohexan/EtOAc = 10:1, R_{f} = 0,23) und anschließender Kugelrohrdestillation (KRD: 221°C,1,3 mbar) erhält man 3,1 g (73 %) Propionsäure-2-[1-(3,3-dimethylcyclohexyl)-1-methylethoxy]-2-oxoethylester als farblose Flüssigkeit.

Geruch: stark moschus, holzig, erogen.
¹H-NMR (200 MHz, CDCl₃): δ (ppm) = 0.80-1,40 (m, 4H), 0,88 (s, 3H), 0,92 (s, 3H), 1,18 (t, J = 7,6 Hz, 3H), 1,42 (d, J = 0,6 Hz, 6H), 1,50-1,76 (m, 4H), 2,03 (tt, J = 12,4, 3,1 Hz, 1H), 2,44 (q, J = 7,6 Hz, 2H), 4,46 (d, J = 15,8 Hz, 1H), 4,55 (d, J = 15,8 Hz, 1H).
¹³C-NMR (50 MHz, CDCl₃): δ (ppm) = 8,9, 22,2, 23,3 (2C), 24,5, 27,0, 27,1, 30,7, 33,6, 39,0, 40,0, 42,0, 61,0, 87,4, 166,7, 173,5.

### Beispiel 10b:

Alternativ zu den Vorschriften in Beispiel 10a, kann Propionsäure-2-[1-(3,3-dimethylcyclohexyl)-1-methylethoxy]-2-oxoethylester über nachfolgenden zweistufigen Syntheseweg hergestellt werden.

Chloressigsäure-1-(3,3-dimethylcyclohexyl)-1-methylethylester: Zu einer auf 0°C abgekühlten Lösung aus 2-(3,3-Dimethylcyclohexyl)-2-propanol (127,7 g, 0,75 mol) in Pyridin (175 ml) wird Chloressigsäureanhydrid (213,7 g, 1,12 mol) portionsweise so zugegeben, dass die Temperatur 10°C nicht übersteigt. Nach beendeter Zugabe lässt man auf Raumtemperatur erwärmen und rührt weitere 3 Stunden. Anschließend kühlt man erneut auf 0°C ab und fügt Wasser (500 ml) hinzu. Die Phasen werden getrennt und die wässrige Phase wird noch dreimal mit Ether (500 ml) extrahiert. Die vereinigten organischen Phasen werden noch zweimal mit 1M HCl gewaschen, nachfolgend getrocknet, abfiltriert und am Rotationsverdampfer von Lösungsmittel befreit. Anschließende Destillation (Sdp. 103°C, 0,5 mbar) liefert 152 g (82 %) Chloressigsäure-1-(3,3-dimethylcyclohexyl)-1-methylethylester als farblose Flüssigkeit.

Propionsäure-2-[1-(3,3-dimethylcyclohexyl)-1-methylethoxy]-2-oxoethylester: Zu einer Lösung aus Propionsäure (1,8 g, 30 mmol) in Aceton (20 ml) gibt man nacheinander K₂CO₃ (6,2 g, 45 mmol) und Chloressigsäure-1-(3,3-dimethylcyclohexyl)-1-methylethylester (2,5 g, 10 mmol). Nachdem man die Suspension 36 Stunden unter Rückfluss erhitzt hat, lässt man abkühlen und gibt Ether (80 ml) und 10 %ige K₂CO₃-Lösung (50 ml) hinzu. Nach erfolgter Phasentrennung befreit man die wässrige Phase von restlichem Aceton und extrahiert die wässrige Phase noch zweimal mit 50 ml Diethylether. Die vereinigten organischen Phasen werden noch je einmal mit Wasser und gesättigter NaCl-Lösung gewaschen, anschließend getrocknet, abfiltriert und am Rotationsverdampfer von Lösungsmittel befreit. Nach flashchromatographischer Reinigung (Cyclohexan/EtOAc = 10:1, R_{f} = 0,23) und anschließender Kugelrohrdestillation (KRD: 215°C, 0,9 mbar) erhält man 2,1 g (78 %) Propionsäure-2-[1-(3,3-dimethylcyclohexyl)-1-methylethoxy]-2-oxoethylester als farblose Flüssigkeit.

Der Geruch und die spektroskopischen Daten sind mit den Daten der Verbindung, die unter Beispiel 10a hergestellt wurde, identisch.

Die nachfolgenden Verbindungen wurden analog der unter Beispiel 8a beschriebenen Vorschriften hergestellt, mit der Änderung, dass man in Beispiel 9 Acetyloxyessigsäure (herstellbar aus Hydroxyessigsäure und Essigsäurechlorid), in Beispiel 10 Isobutyryloxyessigsäure (herstellbar aus Hydroxyessigsäure und Isobuttersäurechlorid) und in Beispiel 11 Butyryloxyessigsäure (herstellbar aus Hydroxyessigsäure und Buttersäurechlorid) in die Veresterung einsetzt. Somit sind an dieser Stelle nur die spektroskopischen Daten aufgeführt:

### Beispiel 11:

### Essigsäure-2-[1-(3,3-dimethylcyclohexyl)-1-methylethoxy]-2-oxoethylester

Geruch: stark moschus, holzig, animalisch.
KRD: 192°C, 0,35 mbar.
¹H-NMR (200 MHz, CDCl₃): δ (ppm) = 0,80-1,08 (m, 3H), 0,88 (s, 3H), 0,92 (s, 3H), 1,30-1,45 (m, 3H), 1,42 (s, 3H), 1,43 (s, 3H), 1,50-1,72 (m, 2H), 2,03 (tt, J = 12,5, 3,1 Hz, 1H), 2,15 (s, 3H), 4,47 (d, J = 15,7 Hz, 1H), 4,52 (d, J = 15,7 Hz, 1H).
¹³C-NMR (50 MHz, CDCl₃): δ (ppm) = 20,5, 22,2, 23,3, 23,4, 24,6, 27,0, 30,7, 33,7, 39,0, 40,1, 42,1, 61,1, 87,6, 166,8, 170,3.

### Beispiel 12:

### Isobuttersäure-2-[1-(3,3-dimethylcyclohexyl)-1-methylethoxy]-2-oxoethylester

Geruch: moschus, ambriert, holzig.
KRD: 261°C, 0.66 mbar.
¹H-NMR (200 MHz, CDCl₃): δ (ppm) = 0,80-1,12 (m, 3H), 0,88 (s, 3H), 0,92 (s, 3H), 1,22 (d, J = 7,0 Hz, 6H), 1,28-1,45 (m, 3H), 1,41 (s, 3H), 1,42 (s, 3H), 1,50-1,75 (m, 2H), 2,05 (tt, J = 12,4, 3,1 Hz, 1H), 2,65 (hep, J = 7,0 Hz, 1H), 4,46 (d, J = 15,8 Hz, 1H), 4,54 (d, J = 15,8 Hz, 1H).
¹³C-NMR (50 MHz, CDCl₃): δ (ppm) = 18,8 (2C), 22,2, 23,3, 23,4, 24,5, 27,0, 30,7, 33,6, 33,7, 39,0, 40,0, 41,9, 60,9, 87,3, 166,7, 176,1.

### Beispiel 13:

### Buttersäure-2-[1-(3,3-dimethylcyclohexyl)-1-methylethoxy]-2-oxoethylester

Geruch: schwach moschus, holzig.
KRD: 274°C, 0,81 mbar.
¹H-NMR (200 MHz, CDCl₃): δ (ppm) = 0,80-1,12 (m, 3H), 0,88 (s, 3H), 0,92 (s, 3H), 0,98 (t, J = 7,4 Hz, 3H), 1,28-1,45 (m, 3H), 1,41 (s, 3H), 1,42 (s, 3H), 1,50-1,75 (m, 2H), 1,70 (sex, J = 7,4 Hz, 2H), 2,04 (tt, J = 12,4, 3,1 Hz, 1H), 2,39 (t, J = 7,4 Hz, 2H), 4,46 (d, J = 15,6 Hz, 1H), 4,54 (d, J = 15,6 Hz, 1H).
¹³C-NMR (50 MHz, CDCl₃): δ (ppm) = 13,6, 18,2, 22,2, 23,3 (2C), 24,5, 27,0, 30,7, 33,6, 35,7, 39,0, 40,0,42,0, 60,9, 87,4, 166,7, 72,7.

Die nachfolgenden Verbindungen wurden analog der unter Beispiel 10a beschriebenen Vorschriften hergestellt, mit der Änderung das als Alkoholkomponente 2-Cyclohexyl-2-propanol, anstatt 2-(3,3-Dimethylcyclohexyl)-2-propanol eingesetzt wurde, welche durch Grignard Reaktion aus Cyclohexylmagnesiumchlorid und Aceton herstellbar ist. In die Veresterung wurden Acetyloxyessigsäure (herstellbar aus Hydroxyessigsäure und Essigsäurechlorid, Beispiel 14) und Propionyloxyessigsäure (herstellbar aus Hydroxyessigsäure und Propionsäurechlorid, Beispiel 15) eingesetzt. Somit sind an dieser Stelle nur die spektroskopischen Daten aufgeführt:

### Beispiel 14:

### Essigsäure-2-(1-cyclohexyl)-1-methylethoxy)-2-oxoethylester

Geruch: schwach moschus, holzig.
KRD: 200°C, 1,3 mbar.
¹H-NMR (200 MHz, CDCl₃): δ (ppm) = 0,90-1,35 (m, 5H), 1,43 (s, 6H), 1,62-1,95 (m, 6H), 2,15 (s, 3H), 4,50 (s, 2H).
¹³C-NMR (50 MHz, CDCl₃): δ (ppm) = 20,4, 23,3 (2C), 26,5, (3C), 27,2 (2C), 46,4, 61,0, 87,5, 166,6, 170,1.

### Beispiel 15:

### Propionsäure-2-(1-cyclohexyl)-1-methylethoxy)-2-oxoethylester

Geruch: schwach moschus, holzige.
KRD: 210°C, 1,2 mbar.
¹H-NMR (200 MHz, CDCl₃): δ (ppm) = 0,90-1,37 (m, 5H), 1,18 (t, J = 7,5 Hz, 3H), 1,43 (s, 6H), 1,62-1,95 (m, 6H), 2,44 (q, J = 7,5 Hz, 2H), 4,50 (s, 2H).
¹³C-NMR (50 MHz, CDCl₃): δ (ppm) = 8,9, 23,3 (2C), 26,4, (3C), 27,1, 27,2 (2C), 46,5, 61,0, 87,5, 166,7, 173,5.

### Beispiel 16 (Vergleichsbeispiel):

### Essigsäure-1-cyclohexyl-1-methylethylester

Zu einer Lösung aus 2-Cyclohexyl-2-propanol (2,1 g, 15,0 mmol) [welches durch Grignard Reaktion aus Cyclohexylmagnesiumchlorid und Aceton erhältlich ist] und Essigsäureanhydrid (4,6 g, 45 mmol) gibt man nacheinander Triethylamin (4,6 g, 45 mmol) und 4-Dimethylaminopyridin (0,40 g, 3,3 mmol). Nach einer Stunde rühren bei Raumtemperatur verdünnt man die Reaktionslösung mit Ether (200 ml) und wäscht die organische Phase je zweimal mit 2 M HCl und gesättigter NaHCO₃-Lösung. Die vereinigten organischen Phasen werden über Na₂SO₄ getrocknet, abfiltriert und am Rotationsverdampfer von Lösungsmittel befreit. Nach Flashchromatographie (Cyclohexan/EtOAc = 20:1, R_{f} = 0,24) und Kugelrohrdestillation (KRD: 120°C, 0,3 mbar) erhält man 2,3 g (83 %) Essigsäure-1-cyclohexyl-1-methylethylester als farbloses Öl.

Geruch: schwach moschus, holzig.
¹H-NMR (200 MHz, CDCl₃): δ (ppm) = 0,90-1,35 (m, 6H), 1,38 (s, 6H), 1,61-1,92 (m, 5H), 1,97 (s, 3H).
¹³C-NMR (50 MHz, CDCl₃): δ (ppm) = 22,4, 23,3 (2C), 26,5 (3C), 27,3 (2C), 46,1, 85,2, 170,2.

Die nachfolgende Verbindung in Beispiel 17 wurde analog der unter Beispiel 16 beschriebenen Vorschrift hergestellt, mit der Änderung, dass Propionsäureanhydrid in die Veresterung eingesetzt wurde. Somit sind an dieser Stelle nur die spektroskopischen Daten aufgeführt:

### Beispiel 17 (Vergleichsbeispiel):

### Propionsäure-1-cyclohexyl-1-methylethylester

Geruch: schwach moschus, holzig.
KRD: 125°C, 0,25 mbar.
¹H-NMR (200 MHz, CDCl₃): δ (ppm) = 0,89-1,36 (m, 6H), 1,09 (t, J = 7,5 Hz, 3H), 1,39 (s, 6H), 1,61-1,96 (m, 5H), 2,24 (q, J = 7,5 Hz, 2H).
¹³C-NMR (50 MHz, CDCl₃): δ (ppm) = 9,3, 23,4 (2C), 26,6 (3C), 27,3 (2C), 28,8, 46,3, 84,8, 173,6.

Die nachfolgenden Verbindungen wurden analog der in Beispiel 16 beschriebenen Vorschrift hergestellt, mit der Änderung das als Alkoholkomponente 2-(3,3-Dimethylcyclohexyl)-2-propanol [welches durch Grignard Reaktion aus Methylmagnesiumchlorid und 1-(3,3-Dimethylcyclohexyl)-ethanon herstellbar ist], anstatt 2-Cyclohexyl-2-propanol, eingesetzt wird. Die Veresterung erfolgte mit Essigsäureanhydrid (Beispiel 18) oder Propionsäureanhydrid (Beispiel 19). Somit sind an dieser Stelle nur die spektroskopischen Daten angegeben:

### Beispiel 18 (Vergleichsbeispiel):

### Essigsäure-1-(3,3-dimethylcyclohexyl)-1-methylethylester

Geruch: moschus, holzig.
KRD: 125°C, 0,17 mbar.
¹H-NMR (200 MHz, CDCl₃): δ (ppm) = 0,8-1,12 (m, 4H), 0,88 (s, 3H), 0,92 (s, 3H), 1,22-1,42 (m, 4H), 1,38 (s, 3H), 1,39 (s, 3H), 1,62-1,76 (m, 1H), 1,97 (s, 3H). ¹³C-NMR (50 MHz, CDCl₃): δ (ppm) = 22,3, 22,4, 23,3, 23,4, 24,6, 27,0, 30,7, 33,7, 39,1, 40,1, 41,6, 84,9, 170,1.

### Beispiel 19 (Vergleichsbeispiel):

### Propionsäure-1-(3,3-dimethylcyclohexyl)-1-methylethylester

Geruch: moschus, holzig.
KRD: 128°C, 0,11 mbar.
¹H-NMR (200 MHz, CDCl₃): δ (ppm) = 0,8-1,13 (m, 4H), 0,88 (s, 3H), 0,91 (s, 3H), 1,09 (t, J = 7,5 Hz, 3H), 1,22-1,42 (m, 4H), 1,38 (s, 3H), 1,39 (s, 3H), 1,58-1,76 (m, 1H), 2,24 (q, J = 7,5 Hz, 2H).
¹³C-NMR (50 MHz, CDCl₃): δ (ppm) = 9,3, 22,4, 23,3, 23,4, 24,6, 27,0, 28,8, 30,7, 33,7, 39,1, 40,2, 41,8, 84,6, 173,4.

### Beispiel 20:

Das vorliegende Parfümöl kann zur Parfümierung diverser kosmetischer Produkte verwendet werden.

### Zusammensetzung:

| Ingredienzien | Gewichtsteile |
|---|---|
| 1. Citrophoral Base (H&R) | 5,0 |
| 2. Aldehyd C10 10% in BA | 5,0 |
| 3. Aldehyd C11 MOA 10% in BA | 3,0 |
| 4. Farenal (H&R) | 3,0 |
| 5. Aldehyd C11 10% in IPM | 5,0 |
| 6. Citroxal 50% in DEP | 2,0 |
| 7. trans Hex-2-enol 10% in BA | 2,0 |
| 8. Vertocitral (H&R) | 1,0 |
| 9. Linalylacetat | 45,0 |
| 10. Citrylal (H&R) | 5,0 |
| 11. Mandarinal (Firmenich) | 4,0 |
| 12. Lilial (Givaudan Roure) | 75,0 |
| 13. Lyral (IFF) | 75,0 |
| 14. Profarnesol (H&R) | 5,0 |
| 15. Nerolidol | 5,0 |
| 16. Linalool | 45,0 |
| 17. Geraniumöl afrikanisch | 5,0 |
| 18. Phenylethylalkohol | 75,0 |
| 19. Geraniol | 15,0 |
| 20. Nerol | 10,0 |
| 21. Hexylzimtaldehyd alpha | 50,0 |
| 22. Methyldihydrojasmonat | 15,0 |
| 23. Benzylsalicylat | 100,0 |
| 24. trans,cis-2-Nonadienol 0,1% in IPM | 5,0 |
| 25. Allylionon (Givaudan Roure) | 3,0 |
| 26. Isomethylionon gamma | 75,0 |
| 27. Eugenol | 7,0 |
| 28. Cedrylacetat | 40,0 |
| 29. Sandolen (H&R) | 5,0 |
| 30. Citral | 5,0 |

| | |
|---|---|
| BA = Benzylalkohol; IPM = Isopropylmyristat; DEP = Diethylphtalat | |

Der Zusatz von
a) 355 Gewichtsteilen Propionsäure-2-(1-cyclohexylethoxy)-2-methylpropylester (Summe 1000 Gewichtsteile) führt zu einer deutlich wahrnehmbaren Harmonisierung der rosig-blumigen Herznote. Darüber hinaus verleiht die feine erogene Moschusnote der vorliegenden Komposition eine hervorragende Strahlung und gesteigerte Haftung. Hierbei setzt sich besonders der wertvolle Charakter von Propionsäure-2-(1-cyclohexylethoxy)-2-methylpropylester im Vergleich zu Kompositionen mit konventionellen Moschusriechstoffen durch.
b) 55 Gewichtsteilen Propionsäure-2-[1-(3,3)-dimethylcyclohexyl)-1-methylethoxy]-2-oxoethylester (Summe 700 Gewichtsteile) verleiht der Komposition eine holzige Moschusnote, die mit existierenden Moschusriechstoffen nicht erreicht wird. Weiterhin gewinnt die gesamte Komposition an Fülle und erscheint wertvoller.

## Patentansprüche

1. Verbindungen der Formel (I) 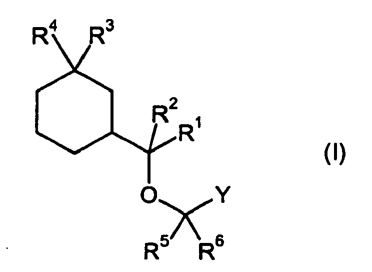 worin
i) R¹ = CH₃, R³ = H oder CH₃ und R² und R⁴ = H sind,
R⁵ und R⁶ -unabhängig voneinander- H oder CH₃ sind, und
Y = -CR⁷R⁸OCOR⁹, wobei R⁷ und R⁸ -unabhängig voneinander- H oder CH₃ sind und
R⁹ eine verzweigte oder unverzweigte C₁ bis C₅ Alkylgruppe oder eine verzweigte oder unverzweigte C₂ bis C₅ Alkenylgruppe ist,
oder
ii) R¹ und R² -unabhängig voneinander- CH₃ oder CH₂CH₃ sind,
R³ und R⁴ -unabhängig voneinander- H oder CH₃ sind,
R⁵ und R⁶ zusammen Sauerstoff sind, und
Y=-CR⁷R⁸OCOR⁹, wobei R⁷, R⁸ und R⁹ die obengenannte Bedeutung haben
oder
iii) R¹ und R² -unabhängig voneinander- CH₃ oder CH₂CH₃ sind,
R³, R⁴, R⁵ und R⁸ -unabhängig voneinander- H oder CH₃ sind, und
Y = -CR⁷R⁸OCOR⁹ ist, wobei R⁷, R⁸ und R⁹ die oben genannte Bedeutung haben.

2. Verbindungen nach Anspruch 1 der Formel (IV) 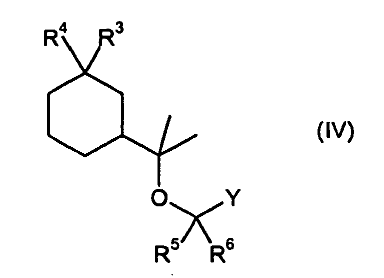 worin
R³ und R⁴ -unabhängig voneinander- H oder CH₃ sind,
R⁵ und R⁶ zusammen Sauerstoff sind, und
Y = -CR⁷R⁸OCOR⁹ ist, wobei R⁷, R⁸ und R⁹ die in Anspruch 1 genannte Bedeutung haben.

3. Verbindungen nach Anspruch 1 der Formel (VI) 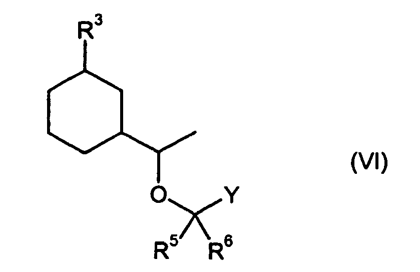 worin
R³ = H oder CH₃ ist,
R⁵ und R⁶ -unabhängig voneinander- H oder CH₃ sind, und
Y = -CR⁷R⁸OCOR⁹ ist, wobei R⁷, R⁸ und R⁹ die in Anspruch 1 genannte Bedeutung haben.

4. Verbindungen nach Anspruch 1, **dadurch gekennzeichnet, dass** es sich um Propionsäure-2-(1-cyclohexylethoxy)-2-methylpropylester, Propionsäure-2-[1-(3,3-dimethylcyclohexyl)-1-methylethoxy]-2-oxoethylester oder Essigsäure-2-[1-(3,3-dimethylcyclohexyl)-1-methylethoxy]-2-oxoethylester handelt.

5. Verfahren zur Herstellung einer Verbindung der Formel (I) 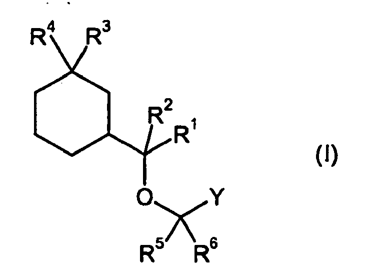 durch Umsetzen eines substituierten Cyclohexanols der Formel 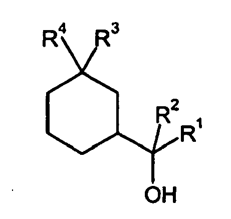 mit einer Carbonsäure der Formel 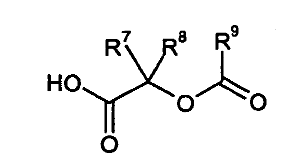 wobei
R¹ und R² -unabhängig voneinander- CH₃ oder CH₂CH₃ sind,
R³ und R⁴ -unabhängig voneinander- H oder CH₃ sind,
R⁵ und R⁶ zusammen Sauerstoff sind, und
Y = -CR⁷R⁸OCOR⁹ ist, wobei R⁷ und R⁸ - unabhängig voneinander - H oder CH₃ sind und R⁹ eine verzweigte oder unverzweigte C₁ bis C₅ Alkylgruppe oder eine verzweigte oder unverzweigte C₂ bis C₅ Alkenylgruppe ist.

6. Verfahren zur Herstellung einer Verbindung der Formel (I) 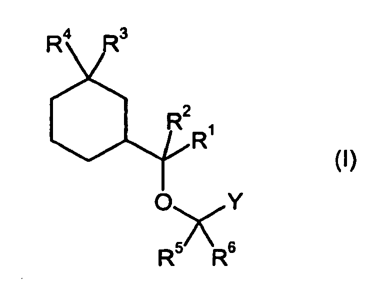 durch Umsetzen eines substituierten Cyclohexanols der Formel 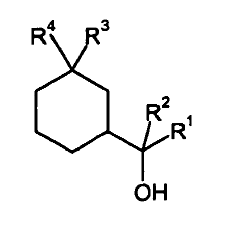 mit einem Epoxid der Formel 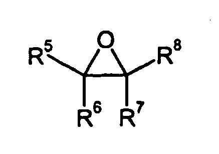 wobei
R¹ = CH₃, R³ = H oder CH₃ und R² und R⁴ = H sind,
R⁵ und R⁶ -unabhängig voneinander- H oder CH₃ sind, und
Y = -CR⁷R⁸OCOR⁹ ist, wobei R⁷ und R⁸ - unabhängig voneinander - H oder CH₃ sind und R⁹ eine verzweigte oder unverzweigte C₁, bis C₅ Alkylgruppe oder eine verzweigte oder unverzweigte C₂ bis C₅ Alkenylgruppe ist
oder
R¹ und R² -unabhängig voneinander- CH₃ oder CH₂CH₃ sind, R³, R⁴, R⁵ und R⁶ -unabhängig voneinander- H oder CH₃ sind, und
Y = -CR⁷R⁸OCOR⁹ ist, wobei R⁷ und R⁸ - unabhängig voneinander - H oder CH₃ sind und R⁹ eine verzweigte oder unverzweigte C₁ bis C₅ Alkylgruppe oder eine verzweigte oder unverzweigte C₂ bis C₅ Alkenylgruppe ist
und nachfolgende Veresterung des Alkohols mit einer Carbonsäure R⁹-COOH oder einen Carbonsäureanhydrid (R⁹-CO)₂O.

7. Verfahren zur Herstellung einer Verbindung der Formel (I) 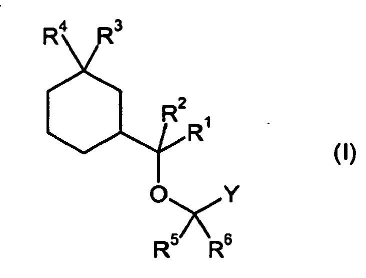 durch Umsetzen eines substituierten Cyclohexanols der Formel 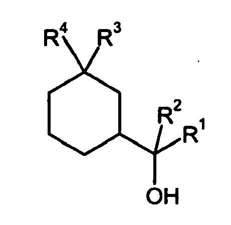 mit einer Carbonsäure XCR⁷R⁸-COOH oder einem Carbonsäureanhydrid (XCR⁷R⁸-CO)₂O in einem ersten Schritt und mit R⁹-COOZ oder (R⁹-CO)₂O in einem zweiten Schritt
wobei
R¹ und R² -unabhängig voneinander- CH₃ oder CH₂CH₃ sind,
R³ und R⁴ -unabhängig voneinander- H oder CH₃ sind,
R⁵ und R⁶ zusammen Sauerstoff sind, und
Y = -CR⁷R⁸OCOR⁹ ist, wobei R⁷ und R⁸ - unabhängig voneinander - H oder CH₃ sind und R⁹ eine verzweigte oder unverzweigte C₁ bis C₅ Alkylgruppe oder eine verzweigte oder unverzweigte C₂ bis C₅ Alkenylgruppe ist
X = Halogen oder OH,
Z = Alkalimetall oder H bedeutet.

8. Verwendung von Verbindungen nach einem der Ansprüche 1 bis 4 als Riechstoffe.

9. Riechstoffmischungen enthaltend Verbindungen nach einem der Ansprüche 1 bis 4.

10. Parfümierte Produkte enthaltend Verbindungen nach einem der Ansprüche 1 bis 4.

## Claims

1. Compounds of the formula (I) 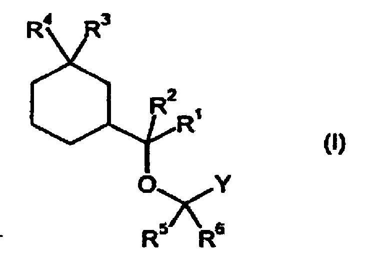 wherein
(i) R¹ = CH₃ , R³ =H or CH₃ and R² and R⁴ = H,
R⁵ and R⁶ -independently of each other- are H or CH₃, and
Y = -CR⁷R⁸OCOR⁹, wherein R⁷ and R⁸ -independently of each other- are H or CH₃ and
R⁹ is a branched or unbranched C₁ to C₅ alkyl group or a branched or unbranched C₂ to C₅ alkenyl group,
or
ii) R¹ and R² -independently of each other- are CH₃ or CH₂CH₃,
R³ and R⁴ -independently of each other- are H or CH₃,
R⁵ and R⁶ together are oxygen, and
Y = -CR⁷R⁸OCOR⁹, wherein R⁷, R⁸ and R⁹ have the abovementioned meaning
or
iii) R¹ and R² -independently of each other- are CH₃ or CH₂CH₃,
R³, R⁴, R⁵ and R⁶ -independently of each other- are H or CH₃, and
Y = -CR⁷R⁸OCOR⁹, wherein R⁷, R⁸ and R⁹ have the abovementioned meaning.

2. Compounds according to claim 1, of the formula (IV) 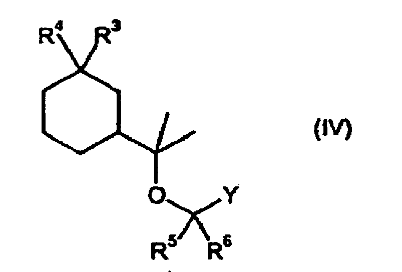 wherein
R³ and R⁴ -independently of each other- are H or CH₃,
R⁵ and R⁶ together are oxygen, and
Y = -CR⁷R⁸OCOR⁹, wherein R⁷, R⁸ and R⁹ have the meaning given in claim 1.

3. Compounds according to claim 1, of the formula (VI) 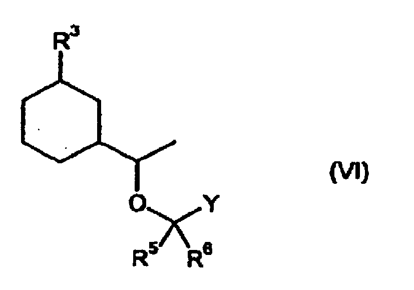 wherein
R³ = H or CH₃,
R⁵ and R⁶ -independently of each other- are H or CH₃, and
Y = -CR⁷R⁸OCOR⁹, wherein R⁷, R⁸ and R⁹ have the meaning given in claim 1.

4. Compounds according to claim 1, **characterized in that** they are propionic acid 2-(1-cyclohexylethoxy)-2-methylpropyl ester, propionic acid 2-[1-(3,3-dimethylcyclohexyl)-1-methylethoxy]-2-oxoethyl ester or acetic acid 2-[1-(3,3-dimethylcyclohexyl)-1-methylethoxy]-2-oxoethyl ester.

5. Process for the preparation of a compound of the formula (I) 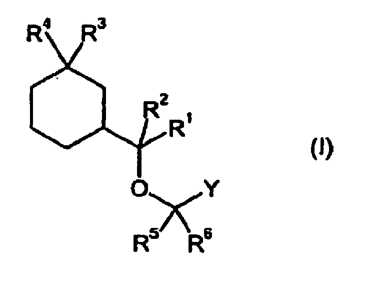 by reaction of a substituted cyclohexanol of the formula 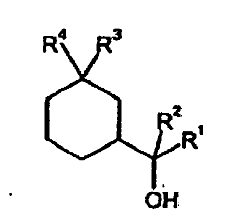 with a carboxylic acid of the formula 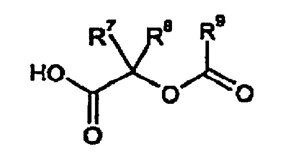 wherein
R¹ and R² -independently of each other- are CH₃ or CH₂CH₃,
R³ and R⁴ -independently of each other- are H or CH₃,
R⁵ and R⁶ together are oxygen, and
Y = -CR⁷R⁸OCOR⁹, wherein R⁷ and R⁸ - independently of each other - are H or CH₃ and R⁹ is a branched or unbranched C₁ to C₅ alkyl group or a branched or unbranched C₂ to C₅ alkenyl group.

6. Process for the preparation of a compound of the formula (I) 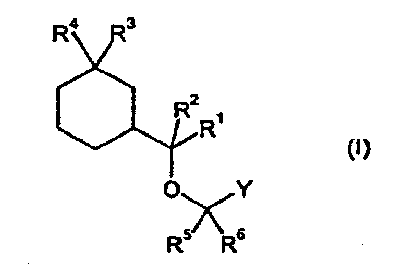 by reaction of a substituted cyclohexanol of the formula 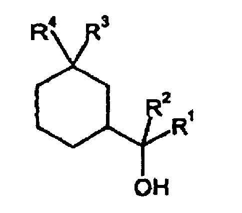 with an epoxide of the formula 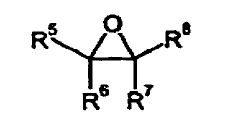 wherein
R¹ = CH₃, R³ = H or CH₃ and R² and R⁴ = H,
R⁵ and R⁶ -independently of each other- are H or CH₃, and
Y = -CR⁷R⁸OCOR⁹, wherein R⁷ and R⁸ - independently of each other - are H or CH₃ and R⁹ is a branched or unbranched C₁ to C₅ alkyl group or a branched or unbranched C₂ to C₅ alkenyl group
or
R¹ and R² -independently of each other- are CH₃ or CH₂H₃,
R³, R⁴, R⁵ and R⁶ -independently of each other- are H or CH₃, and
Y = -CR⁷R⁸OCOR⁹, wherein R⁷ and R⁸ - independently of each other - are H or CH₃ and R⁹ is a branched or unbranched C₁ to C₅ alkyl group or a branched or unbranched C₂ to C₅ alkenyl group
and subsequent esterification of the alcohol with a carboxylic acid R⁹-COOH or a carboxylic acid anhydride (R⁹-CO)₂O.

7. Process for the preparation of a compound of the
formula (I) 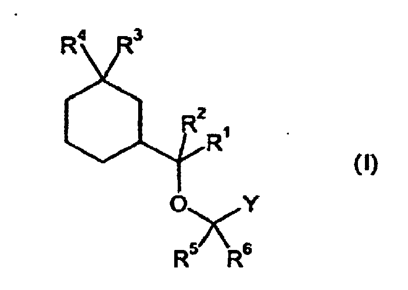 by reaction of a substituted cyclohexanol of the formula 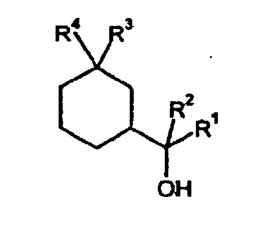 with a carboxylic acid XCR⁷R⁸-COOH or a carboxylic acid anhydride (XCR⁷R⁸-CO)₂ in a first step and with R⁹-COOZ or (R⁹-CO)₂O in a second step
wherein
R¹ and R² -independently of each other- are CH₃ or CH₂CH₃,
R³ and R⁴ -independently of each other- are H or CH₃,
R⁵ and R⁶ together are oxygen, and
Y = -CR⁷R⁸OCOR⁹, wherein R⁷ and R⁸ - independently of each other - are H or CH₃ and R⁹ is a branched or unbranched C₁ to C₅ alkyl or a branched or unbranched C₂ to C₅ alkenyl group
X = halogen or OH,
Z = alkali metal or H.

8. Use of compounds according to one of claims 1 to 4 as odoriferous substances.

9. Odoriferous substance mixtures containing compounds according to one of claims 1 to 4.

10. Perfumed products containing compounds according to one of claims 1 to 4.

## Revendications

1. Composés de la Formule (I): 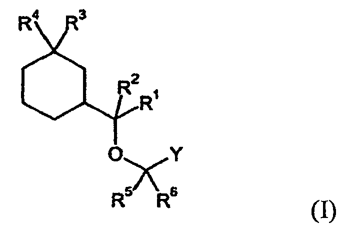 dans laquelle:
i) R¹ = CH₃, R³ = H ou CH₃ et R² et R⁴ = H,
R⁵ et R⁶ sont - indépendamment l'un de l'autre - H ou CH₃, et
Y = -CR⁷R⁸OCOR⁹, où R⁷ et R⁸ sont - indépendamment l'un de l'autre - H ou CH₃, et
R⁹ est un groupe alkyle C₁ à C₅ ramifié ou linéaire ou un groupe alcényle C₂ à C₅ ramifié ou linéaire,
ou
ii) R¹ et R² sont - indépendamment l'un de l'autre - CH₃ ou CH₂CH₃,
R³ et R⁴ sont - indépendamment l'un de l'autre - H ou CH₃,
R⁵ et R⁶ sont ensemble un oxygène, et
Y = -CR⁷R⁸OCOR⁹, où R⁷, R⁸ et R⁹ ont la signification donnée ci-dessus, ou
iii) R¹ et R² sont - indépendamment l'un de l'autre - CH₃ ou CH₂CH₃,
R³, R⁴, R⁵ et R⁶ sont - indépendamment l'un de l'autre - H ou CH₃, et
Y = -CR⁷R⁸OCOR⁹, où R⁷, R⁸ et R⁹ ont la signification donnée ci-dessus.

2. Composés selon la revendication 1 de la Formule (IV): 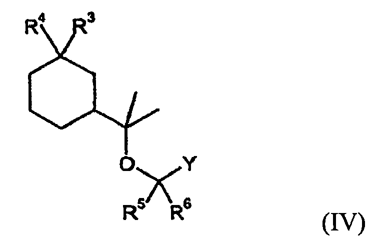 dans laquelle:
R³ et R⁴ sont - indépendamment l'un de l'autre - H ou CH₃,
R⁵ et R⁶ sont ensemble un oxygène, et
Y = -CR⁷R⁸OCOR⁹, où R⁷, R⁸ et R⁹ ont la signification donnée dans la revendication 1.

3. Composés selon la revendication 1 de la Formule (VI): 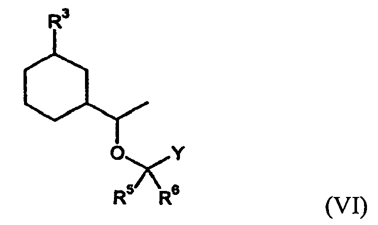 dans laquelle:
R³ = H ou CH₃,
R⁵ et R⁶ sont - indépendamment l'un de l'autre - H ou CH₃, et
Y = -CR⁷R⁸OCOR⁹, où R⁷, R⁸ et R⁹ ont la signification donnée dans la revendication 1.

4. Composés selon la revendication 1, **caractérisés en ce qu'**il s'agit du 2-(1 cyclohexyléthoxy)-2-méthylpropylester d'acide propionique, du 2-[1-(3,3 diméthylcyclohexyl)-1-méthyléthoxy]-2-oxoéthylester d'acide propionique ou du 2-[1-(3,3-diméthylcyclohexyl)-1-méthyléthoxy]-2-oxoéthylester d'acide acétique.

5. Procédé pour former un composé de la Formule (I): 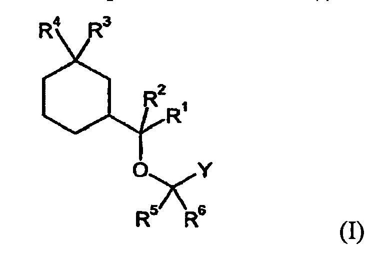 par la réaction d'un cyclohexanol substitué de la Formule: 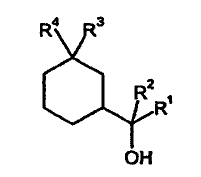 avec un acide carboxylique de la Formule: 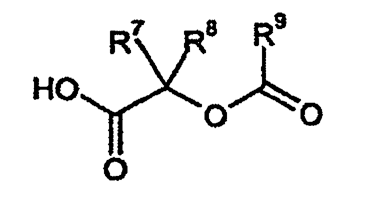 où
R¹ et R² sont - indépendamment l'un de l'autre - CH₃ ou CH₂CH₃,
R³ et R⁴ sont - indépendamment l'un de l'autre - H ou CH₃,
R⁵ et R⁶ sont ensemble un oxygène, et
Y = -CR⁷R⁸OCOR⁹, où R⁷ et R⁸ sont - indépendamment l'un de l'autre - H ou CH₃, et R⁹ est un groupe alkyle C₁ à C₅ ramifié ou linéaire ou un groupe alcényle C₂ à C₅ ramifié ou linéaire.

6. Procédé pour former un composé de la Formule (I): 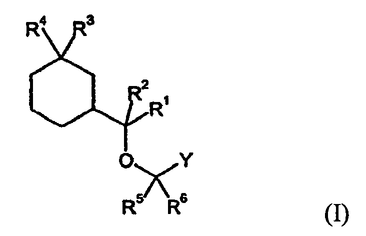 par la réaction d'un cyclohexanol substitué de la Formule: 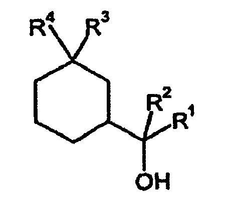 avec un époxyde de la Formule: 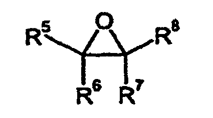 où
R¹ = CH₃, R³ = H ou CH₃ et R² et R⁴ = H,
R⁵ et R⁶ sont - indépendamment l'un de l'autre - H ou CH₃, et
Y = -CR⁷R⁸OCOR⁹, où R⁷ et R⁸ sont - indépendamment l'un de l'autre - H ou CH₃, et R⁹ est un groupe alkyle C₁ à C₅ ramifié ou linéaire ou un groupe alcényle C₂ à C₅ ramifié ou linéaire,
ou
R¹ et R² sont - indépendamment l'un de l'autre - CH₃ ou CH₂CH₃, R³, R⁴, R⁵ et R⁶ sont - indépendamment l'un de l'autre - H ou CH₃, et
Y = -CR⁷R⁸OCOR⁹, où R⁷ et R⁸ sont - indépendamment l'un de l'autre - H ou CH₃, et R⁹ est un groupe alkyle C₁ à C₅ ramifié ou linéaire ou un groupe alcényle C₂ à C₅ ramifié ou linéaire,
et par la suite l'estérification de l'alcool avec un acide carboxylique R⁹-COOH ou un anhydride d'acide carboxylique (R⁹-CO)₂O.

7. Procédé pour former un composé de la Formule (I): 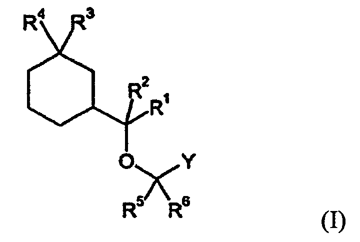 par la réaction d'un cyclohexanol substitué de la Formule: 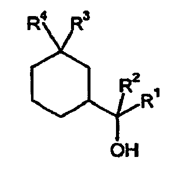 avec un acide carboxylique XCR⁷R⁸-COOH ou un anhydride d'acide carboxylique (XCR⁷R⁸-CO)₂O dans une première étape et avec R⁹-COOZ ou (R⁹-CO)₂O dans une deuxième étape,
où
R¹ et R² sont - indépendamment l'un de l'autre - CH₃ ou CH₂CH₃,
R³ et R⁴ sont - indépendamment l'un de l'autre - H ou CH₃,
R⁵ et R⁶ ensemble sont un oxygène, et
Y = -CR⁷R⁸OCOR⁹, où R⁷ et R⁸ sont - indépendamment l'un de l'autre - H ou CH₃, et R⁹ est un groupe alkyle C₁ à C₅ ramifié ou linéaire ou un groupe alcényle C₂ à C₅ ramifié ou linéaire,
X = halogène ou OH,
Z signifie un métal alcalin ou H.

8. Utilisation d'un composé selon l'une des revendications 1 à 4 en tant que matière odorante.

9. Mélange de matières odorantes comprenant un composé selon l'une des revendications 1 à 4.

10. Produits parfumés comprenant un composé selon l'une des revendications 1 à 4.
